Europäisches Patentamt

European Patent Office

Office européen des brevets

(19)

(11) Veröffentlichungsnummer : **0 253 990 B1**

(12) **EUROPÄISCHE PATENTSCHRIFT**

(45) Veröffentlichungstag der Patentschrift :
**05.06.91 Patentblatt 91/23**

(51) Int. Cl.⁵ : **A61M 5/14**

(21) Anmeldenummer : **87107527.1**

(22) Anmeldetag : **23.05.87**

(54) **Venenkanüle.**

(30) Priorität : 26.05.86 DE 8614252 U
21.01.87 DE 3701585

(43) Veröffentlichungstag der Anmeldung :
27.01.88 Patentblatt 88/04

(45) Bekanntmachung des Hinweises auf die
Patenterteilung :
05.06.91 Patentblatt 91/23

(84) Benannte Vertragsstaaten :
AT BE CH FR GB IT LI LU NL SE

(56) Entgegenhaltungen :
FR-A- 2 171 430
US-A- 3 782 383

(73) Patentinhaber : Saueressig, Ulrich, Dr. med.
Hofaue 91-93
W-5600 Wuppertal 1 (DE)

(72) Erfinder : Saueressig, Ulrich, Dr. med.
Hofaue 91-93
W-5600 Wuppertal 1 (DE)

(74) Vertreter : Patentanwälte Dipl.-Ing. Walter
Kuborn Dipl.-Phys. Dr. Peter Palgen
Mulvanystrasse 2
W-4000 Düsseldorf (DE)

EP 0 253 990 B1

**Beschreibung**

Die Erfindung bezieht sich auf eine Kanüle der dem Oberbegriff des Anspruchs 1 entsprechenden Art.

Einfache Kanülen mit mit Flügeln verschenen Befestigungsstücken sind allgemein bekannt, z.B. aus dem DE-U-85 14 280. Das Kanülenröhrchen steckt in einem Zwischenstück, welches seinerseits in den Schlauch eingesteckt ist und auf welchem das Befestigungsstück mit den seitlichen Flügeln drehbar gelagert ist. Das Kanülenröhrchen steht mit der zugeschärften Spitze etwa 2 cm aus dem Anschlußstück vor. Das konisch sich gegen das Kanülenröhrchen verjüngende Verschlußteil ist an der äußeren Stirnseite des Anschlußstücks vorgesehen.

Das Kanülenröhrchen wird in die Haut und das Blutgefäßt eingestochen und vorgeschoben, bis das Verschlußteil in der Umgebung der Einstichstelle gegen die Hautoberfläche anliegt und das Austreten von Blut neben dem Kanülenröhrchen verhindert. Bei der bekannten Ausführungsform liegt der Verschlußteil nur dann in der gewünschten Weise an der Einstichstelle abdeckend an, wenn das Kanülenröhrchen in seiner gesamten Länge vollständig eingeführt ist. Es ergibt sich also gewissermaßen hierbei eine unveränderliche Einstichlänge. Dies ist jedoch aufgrund unterschiedlicher anatomischer Gegebenheiten nicht in jedem Fall erwünscht. Wenn beispielsweise das zu punktierende Blutgefäß dicht unter der Haut verläuft, wird nur ein Teil der vollen Kanülenlänge benötigt, so daß das Verschlußteil nicht an der Einstichstelle zur Anlage gebracht werden kann und seine Funktion nicht erfüllt. Andererseits gibt es auch Patienten, bei denen die Gefäße relativ tief unter der Haut liegen. In diesem Fall wird zwar die Einstichstelle von dem Verschlußteil abgedeckt, doch reicht die zur Verfügung stehende einführbare Länge des Kanülenröhrchens von etwa 2 cm dann unter Umständen nicht aus, um ein störungsfreies Durchlaufen des Blutes herbeizuführen.

Die vorerwännten Nachteile haften auch der weiterentwickelten Kanüle nach der US-A-3 782 383 an, die dem Oberbegriff des Anspruchs 1 zugrumdeliegt. Bei dieser Kanüle ist das Befestigungsstücke axial verschiebbar auf dem Kanülen röhrchen angeordnet und gegen die Spitze hin eine zusätliche Handhabe vorgeschen, die axial auf dem Kanülen röhrchen festliegt und nur eine unverwänderliche Einstichlänge gestattet.

Der Erfindung liegt die Aufgabe zugrunde, eine Kanüle der dem Oberbegriff entsprechenden Art so auszubilden, daß bei einwandfreier Funktion verschiedene Einstichtiefen möglich sind.

Diese Aufgabe wird durch die in Anspruch 1 wiedergegebene Erfindung gelöst.

Durch die erfindungsgemäße Ausbildung der Kanüle kann eine ausreichende Länge des Kanülenröhrchens gewählt und die Einstichlänge variabel gestaltet und den jeweiligen anatomischen Gegebenheiten des Patienten angepaßt werden. Nach erfolgter Punktion und dem Einschieben des Kanülenröhrchens bis zu der erforderlichen Tiefe kann die Handhabe bis zur Anlage des Verschlußteils an der Hautoberfläche zum Abdichten des Punktionskanals an die Einstichstelle herangeschoben und durch Klebeband auf der Hautoberfläche in dieser Stellung fixiert werden. Die Lage des Kanülenröhrchens zum Blutgefäß, d.h. die Einstichtiefe, wird durch Festkleben des Befestigungsstücks auf der Hautoberfläche aufrechterhalten, so daß sich insgesamt eine sehr sichere Befestigung der Kanüle einstellt.

Eine wichtige Ausgestaltung der Erfindung ist Gegenstand des Anspruchs 2.

Wenn die axiale Fixierung vorgenommen ist, kann die Kanüle an der Handhabe geführt und insbesondere über die Handhabe die zum Einstechen notwendige Kraft aufgebracht werden. An der Handhabe ist dann die Kanüle gut zu erfassen. Wenn dann die axiale Festlegung wieder aufgegeben wird, kann die bis zur Anlage des Verschlußteils an der Einstichstelle erforderliche axiale Verschiebung der Handhabe unter Beibehaltung der erreichten richtigen Einstichlänge erfolgen.

Es empfiehlt sich, daß die Handhabe auch auf dem Kanülenröhrchen drehbar ist, damit auch insoweit eine Anpassungsfähigkeit hinsichtlich der gegenseitigen Lage der Teile gegeben ist (Anspruch 3).

Eine zweckmäßige Ausbildung der Handhabe ist Gegenstand des Anspruchs 4.

Eine vom Aufbau her einfache Weiterbildung dieser Ausbildung, die die Verschiebbarkeit der Handhabe bei gleichzeitiger axialer Festlegbarkeit ergibt, ist in Anspruch 5 wiedergegeben.

Die Verklemmung erfolgt besonders intensiv, wenn die Gestaltung nach Anspruch 6 vorgenommen wird.

Die Ansprüche 7 und 8 sind auf herstellungsmäßig vorteilhafte Detailmerkmale gerichtet, die der einfachen Herstellbarkeit der Handhabe durch Spritzen aus Kunststoff förderlich sind.

In der Zeichnung ist ein Ausführungsbeispiel der Erfindung dargestellt.

Fig. 1 zeigt eine Ansicht der erfindungsgemäßen Kanüle in etwa fünffach vergrößertem Maßstab ;

Fig. 2 zeigt einen Schnitt nach der Linie II-II in Fig. 1 in erneut vergrößertem Maßstab ;

Fig. 3 zeigt eine Fig. 2 entsprechende Ansicht bei hochgeklappten Flügeln der Handhabe.

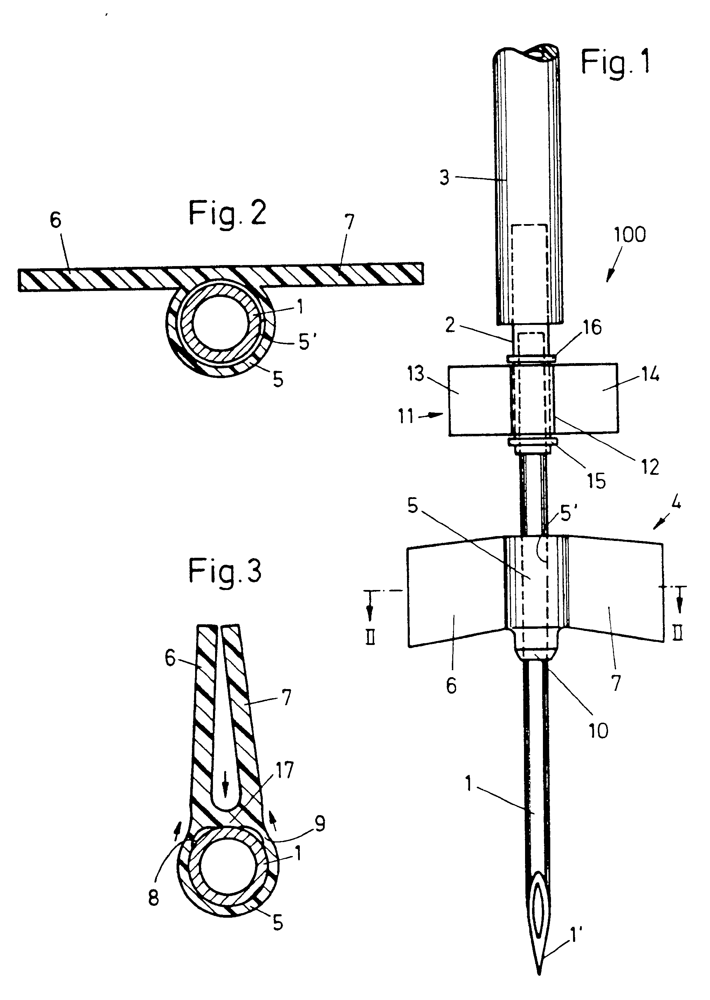

Die als Ganzes mit 100 bezeichnete Kanüle umfaßt ein metallenes Kanülenröhrchen 1, welches an dem in Fig. 1 unteren, zum Einstich bestimmten Ende zu einer Spitze 1' zugeschärft ist. Auf das der Spitze 1' abgewandte Ende des Kanülenröhrchens 1 ist ein Zwischenstück 2 aufgesteckt, welches seinerseits in den Schlauch 3 eingesteckt ist, der beispielsweise zu einem Dialysegerät für die Hämodialyse eines nierenkranken Patienten führt.

Auf dem Zwischenstück 2 sind mit axialem Abstand zwei ringförmige Umfangsvorsprünge 15, 16 vorgesehen, zwischen denen der zylindrische Teil 12 eines als Ganzes mit 11 bezeichneten Befestigungstücks drehbar angeordnet ist. Von dem zylindrischen Teil 12 laden nach beiden Seiten im wesentlichen in einer Ebene verlaufende Flügel 13, 14 aus, die beim Befestigen der Kanüle 100 auf die Hautoberfläche angelegt und mit Klebeband daran befestigt werden.

Auf dem Kanülenröhrchen 1 ist ferner zwischen dem Befestigungsstück 11 und der Spitze 1' eine als Ganzes mit 4 bezeichnete, aus Kunststoff bestehende Handhabe 4 drehbar sowie axial verschiebbar gelagert. Die Handhabe 4 besteht aus einem elastisch verformbaren Kunststoff und umfaßt einen zylindrischen Teil 5 mit einer zylindrischen Ausnehmung 5', die von dem Kanulenröhrchen 1 mit geringem Spiel durchgriffen wird, so daß die Handhabe 4 auf dem Kanülenröhrchen 1 einen leichten Schiebesitz aufweist und ohne großen Kraftaufwand in Längsrichtung verlagert und verdreht werden kann. Die Handhabe 4 soll aber nicht lose auf dem Kanülenröhrchen 1 sitzen, so daß sie von selbst abfällt.

Der zylindrische Teil 5 der Handhabe 4 weist auf der der Spitze 1' zugewandten Seite einen ringförmigen, sich konisch gegen den Außenumfang des Kanülenröhrchens 1 verjüngenden Verschlußteil 10 auf, der dazu bestimmt ist, gegen die Einstichstelle der Kanüle 100 angeschoben zu werden und diese gegen den Austritt von Blut neben dem Kanülenröhrchen 1 abzudichten.

Die Handhabe 4 weist ferner zwei im normalen kräftefreien Zustand etwa in einer Ebene liegende Flügel 6, 7 auf, deren Breite mit der Länge des zylindrischen Teils 5 etwa übereinstimmt und die nach jeder Seite von dem zylindrischen Teil 5 um etwa den gleichen Betrag ausladen.

Wie aus Fig. 2 ersichtlich ist, liegen die Flügel 6, 7 in einer zu dem zylindrischen Teil 5 etwa tangentialen Ebene. In dem in Fig. 2 wiedergegebenen kräftefreien Zustand weist das Kanülenröhrchen 1 in der Ausnehmung 5' des zylindrischen Teils 5 den erwähnten leichten Schiebesitz auf. Das Spiel zwischen dem Außenumfang des Kanülenröhrchens 1 und der Ausnehmung 5' ist in Fig. 2 übertrieben dargestellt.

Wenn die Flügel 6, 7 jedoch in der aus Fig. 3 ersichtlichen Weise hochgebogen und gegeneinandergeschwenkt werden, wird der zylindrische Teil verformt, so daß die Ausnehmung 5' nicht mehr kreisrund ist. In den Bereichen 8, 9 treten Zugspannungen auf, wie es durch die Pfeile angedeutet ist, so daß der in Fig. 3 untere Bereich des zylindrischen Teils 5 fest gegen den Außenumfang des Kanülenröhrchens 1 gezogen wird. In dem mittleren Bereich 17 jedoch tritt ein Druck in der entgegengesetzten Richtung auf, der durch den Pfeil zwischen den Flügeln 6, 7 angedeutet ist und zu einer Anlage des Bereichs 17 an dem Außenumfang des Kanülenröhrchens 1 führt. Der leichte Schiebesitz ist aufgehoben, und es ist die Handhabe 4 auf dem Kanülenröhrchen 1 festgeklemmt. Werden die Flügel 6, 7 freigegeben, so stellen sie sich im wesentlichen in die in Fig. 2 wiedergegebene Lage zurück, wodurch der leichte Schiebesitz wiederhergestellt werden kann.

Zur Durchführung der Punktion mit der Kanüle 100 werden die beiden Flügel 6 und 7 der Handhabe 4 manuell erfaßt und gegeneinandergedrückt, wodurch die geschilderte kraftschlüssige Verbindung zwischender Handhabe 4 und dem Kanülenröhrchen 1 herbeigefüht wird, so daß sich diese in der erforderlichen Länge in das Körpergefäß des Patienten einschieben läßt. In der auf diese Weise hergestellten Funktionsstellung der Kanüle 100 wird durch Spreizen der beiden Flügel 6 und 7 der Kraftschluß wieder aufgehoben und die Handhabe 4 auf dem Kanülenröhrchen 1 so weit vorgeschoben, bis der Verschlußteil 10 an der Einstichstelle dichtend anliegt. Damit die Kanüle 100 diese Lage für die Dauer des Blutdurchlaufs zuverlässig beibehält, ohne daß sie sich in Längs- oder Querrichtung verschiebt, werden sowohl die beiden Flügel 6 und 7 der Handhabe 4 als auch die beiden Flügel 13 und 14 des Befestigungsstücks 11 mittels geeigneter Klebebänder wie Heftpflaster auf der Haut des Patienten fixiert.

## Ansprüche

1. Kanüle (100) zur Herstellung eines äußeren Anschlusses zu einem Körpergefäß, insbesondere zum Blutkreislauf bei der Hämodialyse,

mit einem vorn zum Einstechen in ein Körpergefäß zu einer Spitze (1') zugeschärften Kanülenröhrchen (1), welches am hinteren Ende in einen Anschlußschlauch (3) oder dergleichen mündet, mit einem nahe dem hinteren Ende auf dem Kanülenröhrchen angeordneten Befestigungsstück (11) mit seitlichen Flügeln (13, 14), die mittels Klebeband auf der Hautoberfläche festlegbar sind,

und mit einer auf dem Kanülenröhrchen (1) zwischen dem Befestigungsstück (11) und der Spitze (1') des Kanülenröhrchens (1) angeordneten Handhabe (4), die auf ihrer der Spitze (1') des Kanülenröhrchens (1) zugewandten Seite das Kanülenröhrchen (1) ringförmig umgibt und sich mit der Stirnseite konisch gegen das Kanülenröhrchen (1) verjüngt, dadurch gekennzeichnet, daß das Befestigungsstück (11) auf dem Kanülenröhrchen (1) axial fixiert ist und daß die Handhabe (4) auf dem Kanülenröhrchen (1) bis zur dichtenden Anlage des sich konisch gegen das Kanülenröhrchen verjüngende Verschlußteils (10) an der Einstichstelle gegen die Einstichstelle anschiebbar ist.

2. Kanüle nach Anspruch 1, dadurch gekennzeichnet, daß die Handhabe (4) wahlweise axial auf dem Kanülenröhrchen (1) festlegbar ist.

3. Kanüle nach Anspruch 1 oder 2, dadurch gekennzeichnet, daß die Handhabe (4) auf dem Kanülenröhrchen (1) auch drehbar ist.

4. Kanüle nach einem der Ansprüche 1 bis 3, dadurch gekennzeichnet, daß die Handhabe (4) eine das Kanülenröhrchen (1) mit Schiebesitz umgreifende Ausnehmung (5') aufweist.

5. Kanüle nach Anspruch 4, dadurch gekennzeichnet, daß die Ausnehmung (5') in einem etwa zylindrischen Teil (5) aus verformbarem Material mit seitlichen Flügeln (6, 7) angeordnet ist und der Schiebesitz bei seitlich ausgestreckten Flügeln (6, 7) gegeben ist, während durch Gegeneinanderklappen der Flügel (6, 7) um eine zur Achse des zylindrischen Teils (5) parallele Achse eine Verformung des zylindrischen Teils (5) unter Verklemmung des Kanülenröhrchens (1) in der Ausnehmung (5') eintritt.

6. Kanüle nach Anspruch 5, dadurch gekennzeichnet, daß die Flügel (6, 7) etwa in einer zum zylindrischen Teil (5) tangentialen Ebene verlaufen.

7. Kanüle nach Anspruch 5 oder 6, dadurch gekennzeichnet, daß die Flügel (6, 7) an den zylindrischen Teil (5) einstückig angeformt sind.

8. Kanüle nach einem der Ansprüche 5 bis 7, dadurch gekennzeichnet, daß das Verschlußteil (10) einstückig an den zylindrischen Teil (5) angeformt ist.

## Claims

1. Cannula (100) for producing an external connection to an organ in the body, in particular to the blood circulation system in haemodialysis, with a distal cannula needle (1) sharpened to a point (1') for inserting in an organ of the body, the needle opening at its rear end into a connecting tube (3) or the like,

with an attachment piece (11) mounted on the cannula needle near the rear end, with lateral wings (13, 14) which can be secured by means of adhesive tape to the surface of the skin, and with a handle (4) mounted on the needle (1) between the attachment piece (11) and the tip (1') of the needle (1), the handle annularly surrounding the needle (1) on that part of it which is towards the tip (1') of the needle (1), and the handle having its end portion tapering conically towards the cannula needle (1) characterised in that, the attachment piece (11) is axially fixed on the cannula needle (1) and the handle (4) can be displaced along the cannula needle (1) to the point where the closure portion (10) conically tapering inwards against the against the needle comes into sealing engagement against the point of insertion.

2. Cannula according to claim 1 characterised in that the handle (4) can at will be located axially on the needle (1).

3. Cannula according to claim 1 or 2 characterised in that the handle (4) is also rotatable on the cannula needle (1).

4. Cannula according to one of claims 1 to 3 characterised in that the handle (4) has an opening (5') embracing the cannula needle (1) with a sliding engagement.

5. Cannula according to claim 4 characterised in that the opening (5') is arranged in a substantially cylindrical portion (5) of deformable material having lateral wings (6, 7) and the sliding engagement is obtained with the wings (6, 7) outstretched laterally, whilst by folding the wings (6, 7) against one another about an axis parallel to the axis of the cylindrical portion (5) there is deformation of the cylindrical portion (5) with a clamping action on the cannula needle (1) in the opening (5'),

6. Cannula according to claim 5 characterised in that the wings (6, 7) extends substantially in a plane which is tangential to the cylindrical portion (5).

7. Cannula according to claim 5 or 6 characterised in that the wings (6, 7) are formed integrally on the cylindrical portion (5).

8. Cannula according to one of claims 5 to 7 characterised in that the closure portion (10) is formed integrally with the cylindrical portion (5).

## Revendications

1. Canule (100) destinée à constituer un raccordement externe avec un vaisseau sanguin, notamment pour un recyclage sanguin, en cas d'hémodialyse, avec une aiguille de canule (1) acérée au niveau d'une pointe (1') faite pour être piquée dans un vaisseau sanguin, cette aiguille débouchent du côté arrière dans un tuyau flexible de raccordement (3) ou quelque chose de semblable, avec une pièce de fixation (11) placée à proximité de l'extrémité arrière de l'aiguille de canule, munie de battants latéraux (13, 14), qui peuvent être fixés à la surface de la peau à l'aide de bande adhésive, et avec une poignée (4) placée sur l'aiguille de canule (1) entre la pièce de fixation (11) et la pointe (1') de l'aiguille de canule (1), qui entoure de façon annulaire l'aiguille de canule (1) sur son côté opposé à la pointe (1') de l'aiguille de canule (1), et qui s'effile sur son côté latéral, de façon conique, contre l'aiguille de canule (1), caractérisée en ce que, la pièce de fixation (11) est fixée dans l'axe de l'aiguille de canule (1) et en ce que la poignée (4) peut être poussée sur l'aiguille de canule (1) jusqu'à l'endroit assurant l'étanchéité de la pièce d'étanchéité (10) s'effilant de façon conique le long de l'aiguille de canule, au niveau du point de piqûre à proximité immédiate du point de piqûre.

2. Canule selon la revendication 1, caractérisée en ce que la poignée (4) peut être fixée facultativement dans l'axe de l'aiguille de canule (1).

3. Canule selon l'une au moins des revendications 1 ou 2, caractérisée en ce que la poignée (4) peut pivoter aussi autour de l'aiguille de canule.

4. Canule selon l'une quelconque des revendications 1 à 3, caractérisée en ce que la poignée (4) présente un évidement (5') entourant l'aiguille de canule d'un siège coulissant.

5. Canule selon la revendication 4, caractérisée en ce que l'évidement (5') est aménagé dans une partie à peu près cylindrique, faite en matériau déformable, munie de battants latéraux (6, 7), et le siège coulissant y est placé lorsque les battants (6, 7) sont déployés latéralement, tandis que, par le rabattement des battants (6, 7) sur un axe parallèle à celui de la pièce cylindrique (5), une déformation de la pièce cylindrique (5) provoque le blocage de l'aiguille de canule (1) dans l'évidement (5').

6. Canule selon la revendication 5, caractérisée en ce que les battants (6, 7) sont situés à peu près dans un plan tangentiel à la pièce cylindrique.

7. Canule selon l'une au moins des revendications 5 ou 6, caractérisée en ce que les battants (6, 7) sont formés d'un seul tenant par rapport à la pièce cylindrique (5).

8. Canule selon l'une au moins des revendications 5 à 7, caractérisée en ce que la pièce d'étanchéité (10) est formée d'un seul tenant par rapport à la pièce cylindrique (5).

Fig. 1

Fig. 2

Fig. 3